# EUROPEAN PATENT APPLICATION

(11) **EP 4 119 160 A1**
(43) Date of publication of application: **18.01.2023**
(21) Application number: 21768434.9
(22) Date of filing: 11.03.2021
(51) Int. Cl.: A61K 39/395, A61K 45/00, A61P 35/00, A61P 37/02, A61P 43/00, C07K 16/30

(54) **MEDICAMENT FOR TREATMENT AND/OR PREVENTION OF CANCER**

(30) Priority: 12.03.2020 JP 2020043022
(71) Applicant: Toray Industries, Inc., Tokyo 103-8666 (JP)
(72) Inventor: OKANO Fumiyoshi, Kamakura-shi, Kanagawa 248-8555 (JP)
(74) Representative: Mewburn Ellis LLP
(86) International application number: PCT/JP2021/009799
(87) International publication number: WO 2021/182574

(57) **Abstract**

An object of the present invention is to provide a medicament for treatment and/or prevention of cancer. In order to attain the object, the present invention provides a medicament for treatment and/or prevention of cancer, comprising an antibody or a fragment thereof having an immunological reactivity with CAPRIN-1 protein, and an immune checkpoint inhibitor together or separately in combination.

## Description

### Technical Field

The present invention relates to a medicament for treatment and/or prevention of cancer, using an antibody against CAPRIN-1 protein, or a fragment thereof, and an immune checkpoint inhibitor.

### Background Art

Various antibody medicines targeting specific antigen proteins on cancer cells are applied as therapeutic agents for cancers with fewer side effects to cancer treatment because of their cancer specificity. For example, cytoplasmic-activation and proliferation-associated protein 1 (CAPRIN-1) is expressed on cell membrane surfaces of many solid cancers. Antibodies against this CAPRIN-1 protein are known to be promising in pharmaceutical uses for treatment and/or prevention of cancers (Patent Literature 1).

In recent years, treatment methods using combinations of pluralities of therapeutic agents for cancer have been clinically used as standard treatment methods in order to enhance the effectiveness of the therapeutic agents for cancers. It has been performed in common to treat using a plurality of anticancer agents, for example, colon cancer is treated by a treatment method using a combination of irinotecan, folinic acid, and fluorouracil; breast cancer is treated by a treatment method using a combination of doxorubicin and cyclophosphamide or a combination of paclitaxel, trastuzumab, and pertuzumab; and gastric cancer is treated using a plurality of anticancer agents such as carboplatin and fluorouracil. Therapeutic agents for cancers comprising anti-CAPRIN-1 antibodies as active ingredients have also been confirmed to have superior therapeutic effects on the cancers by combinations with chemotherapeutics (Patent Literature 2). However, treatment of a cancer by a combined use of chemotherapeutics is not effective for every cancer to which the treatment is applied, and few combinations of chemotherapeutics synergistically or drastically enhance therapeutic effects, though some combinations additively enhance therapeutic effects.

In the case of malignant melanoma patients, for example, the median survival time of patients with distal metastasis to the skin, lymph node, and/or gastrointestinal tract is only 12.5 months (their 5-year survival rate is only 14%); the median survival time of patients with metastasis to the lung is 8.3 months (their 5-year survival rate is 4%); and the median survival time of patients with metastasis to the liver, brain, and/or bone is 4.4 months (their 5-year survival rate is 3%). Thus, prognosis is very poor. Application of partial resection, etc. of the site of metastasis is limited to patients having a good general condition (Non Patent Literature 1).

In recent years, so-called immune checkpoint inhibitors which treat cancer by enhancing or complementing immunity against cancer *in vivo* have been approved or clinically tested for many cancers.

For example, a phase III trial targeting a combination of two or more types of immune checkpoints (CheckMate-067 trial) has been conducted, and the first-line treatment of advanced metastatic malignant melanoma by combination therapy of nivolumab and ipilimumab has produced an effect of 52% in terms of the proportion of patients who survived for 5 years or longer (5-year survival rate) (Non Patent Literature 2).

The objective response rate (ORR) of a group given a combination of nivolumab and ipilimumab for untreated advanced/metastatic renal cell carcinoma has been 41% and thus, improved response has been confirmed in this group, whereas ORR has been 34% in a group given Sutent alone as a control group.

### Citation List

### Patent Literature

Patent Literature 1: WO2010/016526
Patent Literature 2: WO2011/096535

### Non Patent Literature

Non Patent Literature 1: J Am Coll Surg, 1995, 181, 193-201
Non Patent Literature 2: The New England Journal of Medicine, 2019, 381, 1535-1546

### Summary of Invention

### Technical Problem

An object of the present invention is to provide a medicament for treatment and/or prevention of cancer specifically expressing CAPRIN-1 protein on a cell surface.

### Solution to Problem

As a result of intensive studies, the present inventors have found that a combination of an antibody against CAPRIN-1 protein, or a fragment thereof, having an immunological reactivity with cancer cells, and an immune checkpoint inhibitor exerts a very strong antitumor effect on a cancer patient, in particular, a cancer patient with a previous history of cancer treatment with an immune checkpoint inhibitor. On the basis of these findings, the present invention has been completed.

Specifically, the present invention relates to the following embodiments (1) to (18):

(1) A medicament for treatment and/or prevention of cancer, comprising an antibody or a fragment thereof having an immunological reactivity with CAPRIN-1 protein, and an immune checkpoint inhibitor together or separately in combination.
(2) The medicament according to (1), wherein the immune checkpoint inhibitor is a drug that inhibits interaction between PD-1 and PD-L1 and/or a drug that inhibits interaction between CTLA-4 and CD80 or CD86.
(3) The medicament according to (1) or (2), wherein the immune checkpoint inhibitor is an anti-PD-1 antibody, an anti-PD-L1 antibody and/or an anti-CTLA-1 antibody.
(4) The medicament according to any of (1) to (3), wherein the immune checkpoint inhibitor is nivolumab.
(5) The medicament according to any of (1) to (4), wherein the cancer is cancer in a cancer patient who has not responded to cancer treatment with a medicament other than cancer treatment with a medicament comprising an antibody or a fragment thereof having an immunological reactivity with CAPRIN-1 protein, and an immune checkpoint inhibitor together or separately in combination.
(6) The medicament according to any of (1) to (5), wherein the cancer is cancer in a cancer patient who has not responded to cancer treatment with an immune checkpoint inhibitor.
(7) The medicament according to any of (1) to (6), wherein the antibody or the fragment thereof has an immunological reactivity with CAPRIN-1 protein having an amino acid sequence shown in any one of the even numbered SEQ ID NOs: 2 to 30, or an amino acid sequence having 80% or more sequence identity with the amino acid sequence.
(8) The medicament according to any of (1) to (7), wherein the antibody or the fragment thereof has an immunological reactivity with an extracellular region of a CAPRIN-1 protein present on a cancer cell surface.
(9) The medicament according to any of (1) to (8), wherein the antibody or the fragment thereof has an immunological reactivity with a partial polypeptide of CAPRIN-1 protein, the partial polypeptide having an amino acid sequence represented by any one of SEQ ID NOs: 31 to 35, 296 to 299, 308 and 309, or an amino acid sequence having 80% or more sequence identity with the amino acid sequence.
(10) The medicament according to any of (1) to (9), wherein the antibody is a monoclonal antibody or a polyclonal antibody.
(11) The medicament according to any of (1) to (10), wherein the antibody or the fragment thereof is any one of the following (A) to (M):
   (A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
   (B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
   (C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
   (D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
   (E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
   (F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
   (G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
   (H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
   (I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
   (J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
   (K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
   (L) an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein; and
   (M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.
(12) The medicament according to any of (1) to (11), wherein the antibody or the fragment thereof is any one of the following (a) to (al):
   (a) an antibody or a fragment thereof comprising a heavy-chain variable region comprising the amino acid sequence of SEQ ID NO: 39 and a light-chain variable region comprising the amino acid sequence of SEQ ID NO: 43;
   (b) an antibody or a fragment thereof comprising a heavy-chain variable region comprising the amino acid sequence of SEQ ID NO: 47 and a light-chain variable region comprising the amino acid sequence of SEQ ID NO: 51;
   (c) an antibody or a fragment thereof comprising a heavy-chain variable region comprising the amino acid sequence of SEQ ID NO: 55 and a light-chain variable region comprising the amino acid sequence of SEQ ID NO: 59;
   (d) an antibody or a fragment thereof comprising a heavy-chain variable region comprising the amino acid sequence of SEQ ID NO: 63 and a light-chain variable region comprising the amino acid sequence of SEQ ID NO: 67;
   (e) an antibody or a fragment thereof comprising a heavy-chain variable region comprising the amino acid sequence of SEQ ID NO: 68 and a light-chain variable region comprising the amino acid sequence of SEQ ID NO: 69;
   (f) an antibody or a fragment thereof comprising a heavy-chain variable region comprising the amino acid sequence of SEQ ID NO: 70 and a light-chain variable region comprising the amino acid sequence of SEQ ID NO: 71;
   (g) an antibody or a fragment thereof comprising a heavy-chain variable region comprising the amino acid sequence of SEQ ID NO: 72 and a light-chain variable region comprising the amino acid sequence of SEQ ID NO: 73;
   (h) an antibody or a fragment thereof comprising a heavy-chain variable region comprising the amino acid sequence of SEQ ID NO: 74 and a light-chain variable region comprising the amino acid sequence of SEQ ID NO: 75;
   (i) an antibody or a fragment thereof comprising a heavy-chain variable region comprising the amino acid sequence of SEQ ID NO: 76 and a light-chain variable region comprising the amino acid sequence of SEQ ID NO: 77;
   (j) an antibody or a fragment thereof comprising a heavy-chain variable region comprising the amino acid sequence of SEQ ID NO: 78 and a light-chain variable region comprising the amino acid sequence of SEQ ID NO: 79;
   (k) an antibody or a fragment thereof comprising a heavy-chain variable region comprising the amino acid sequence of SEQ ID NO: 80 and a light-chain variable region comprising the amino acid sequence of SEQ ID NO: 81;
   (l) an antibody or a fragment thereof comprising a heavy-chain variable region comprising the amino acid sequence of SEQ ID NO: 82 and a light-chain variable region comprising the amino acid sequence of SEQ ID NO: 83;
   (m) an antibody or a fragment thereof comprising a heavy-chain variable region comprising the amino acid sequence of SEQ ID NO: 84 and a light-chain variable region comprising the amino acid sequence of SEQ ID NO: 85;
   (n) an antibody or a fragment thereof comprising a heavy-chain variable region comprising the amino acid sequence of SEQ ID NO: 86 and a light-chain variable region comprising the amino acid sequence of SEQ ID NO: 87;
   (o) an antibody or a fragment thereof comprising a heavy-chain variable region comprising the amino acid sequence of SEQ ID NO: 88 and a light-chain variable region comprising the amino acid sequence of SEQ ID NO: 89;
   (p) an antibody or a fragment thereof comprising a heavy-chain variable region comprising the amino acid sequence of SEQ ID NO: 90 and a light-chain variable region comprising the amino acid sequence of SEQ ID NO: 91;
   (q) an antibody or a fragment thereof comprising a heavy-chain variable region comprising the amino acid sequence of SEQ ID NO: 92 and a light-chain variable region comprising the amino acid sequence of SEQ ID NO: 93;
   (r) an antibody or a fragment thereof comprising a heavy-chain variable region comprising the amino acid sequence of SEQ ID NO: 94 and a light-chain variable region comprising the amino acid sequence of SEQ ID NO: 95;
   (s) an antibody or a fragment thereof comprising a heavy-chain variable region comprising the amino acid sequence of SEQ ID NO: 96 and a light-chain variable region comprising the amino acid sequence of SEQ ID NO: 97;
   (t) an antibody or a fragment thereof comprising a heavy-chain variable region comprising the amino acid sequence of SEQ ID NO: 98 and a light-chain variable region comprising the amino acid sequence of SEQ ID NO: 99;
   (u) an antibody or a fragment thereof comprising a heavy-chain variable region comprising the amino acid sequence of SEQ ID NO: 100 and a light-chain variable region comprising the amino acid sequence of SEQ ID NO: 101;
   (v) an antibody or a fragment thereof comprising a heavy-chain variable region comprising the amino acid sequence of SEQ ID NO: 102 and a light-chain variable region comprising the amino acid sequence of SEQ ID NO: 103;
   (w) an antibody or a fragment thereof comprising a heavy-chain variable region comprising the amino acid sequence of SEQ ID NO: 104 and a light-chain variable region comprising the amino acid sequence of SEQ ID NO: 105;
   (x) an antibody or a fragment thereof comprising a heavy-chain variable region comprising the amino acid sequence of SEQ ID NO: 106 and a light-chain variable region comprising the amino acid sequence of SEQ ID NO: 107;
   (y) an antibody or a fragment thereof comprising a heavy-chain variable region comprising the amino acid sequence of SEQ ID NO: 108 and a light-chain variable region comprising the amino acid sequence of SEQ ID NO: 109;
   (z) an antibody or a fragment thereof comprising a heavy-chain variable region comprising the amino acid sequence of SEQ ID NO: 110 and a light-chain variable region comprising the amino acid sequence of SEQ ID NO: 111;
   (aa) an antibody or a fragment thereof comprising a heavy-chain variable region comprising the amino acid sequence of SEQ ID NO: 112 and a light-chain variable region comprising the amino acid sequence of SEQ ID NO: 113;
   (ab) an antibody or a fragment thereof comprising a heavy-chain variable region comprising the amino acid sequence of SEQ ID NO: 114 and a light-chain variable region comprising the amino acid sequence of SEQ ID NO: 115;
   (ac) an antibody or a fragment thereof comprising a heavy-chain variable region comprising the amino acid sequence of SEQ ID NO: 116 and a light-chain variable region comprising the amino acid sequence of SEQ ID NO: 117;
   (ad) an antibody or a fragment thereof comprising a heavy-chain variable region comprising the amino acid sequence of SEQ ID NO: 118 and a light-chain variable region comprising the amino acid sequence of SEQ ID NO: 119;
   (ae) an antibody or a fragment thereof comprising a heavy-chain variable region comprising the amino acid sequence of SEQ ID NO: 120 and a light-chain variable region comprising the amino acid sequence of SEQ ID NO: 121;
   (af) an antibody or a fragment thereof comprising a heavy-chain variable region comprising the amino acid sequence of SEQ ID NO: 122 and a light-chain variable region comprising the amino acid sequence of SEQ ID NO: 123;
   (ag) an antibody or a fragment thereof comprising a heavy-chain variable region comprising the amino acid sequence of SEQ ID NO: 124 and a light-chain variable region comprising the amino acid sequence of SEQ ID NO: 125;
   (ah) an antibody or a fragment thereof comprising a heavy-chain variable region comprising the amino acid sequence of SEQ ID NO: 126 and a light-chain variable region comprising the amino acid sequence of SEQ ID NO: 127;
   (ai) an antibody or a fragment thereof comprising a heavy-chain variable region comprising the amino acid sequence of SEQ ID NO: 128 and a light-chain variable region comprising the amino acid sequence of SEQ ID NO: 129;
   (aj) an antibody or a fragment thereof comprising a heavy-chain variable region comprising the amino acid sequence of SEQ ID NO: 130 and a light-chain variable region comprising the amino acid sequence of SEQ ID NO: 131;
   (ak) an antibody or a fragment thereof comprising a heavy-chain variable region comprising the amino acid sequence of SEQ ID NO: 132 and a light-chain variable region comprising the amino acid sequence of SEQ ID NO: 133; and
   (al) an antibody or a fragment thereof comprising a heavy-chain variable region comprising the amino acid sequence of SEQ ID NO: 300 and a light-chain variable region comprising the amino acid sequence of SEQ ID NO: 304.
(13) The medicament according to any of (1) to (12), wherein the antibody is a human antibody, a humanized antibody, a chimeric antibody or a single chain antibody.
(14) The medicament according to any of (1) to (13), wherein the cancer is cancer expressing CAPRIN-1 protein on a cell membrane surface.
(15) The medicament according to any of (1) to (14), wherein the cancer is melanoma, breast cancer, kidney cancer, colon cancer, pancreatic cancer, lung cancer, renal cell carcinoma, Hodgkin's lymphoma, head and neck cancer, gastric cancer, mesothelioma, colorectal cancer, esophageal cancer, gastroesophageal junction cancer, hepatocellular carcinoma, glioblastoma, urothelial carcinoma, ovarian cancer, urinary bladder cancer, uterine cancer, primary central nervous system lymphoma, primary testicular lymphoma, biliary tract cancer, bile duct cancer, brain tumor, prostate cancer, leukemia, lymphoma, liver cancer, sarcoma, fibrosarcoma, mastocytoma, adrenocortical carcinoma, Ewing's tumor, multiple myeloma, testicular cancer, thyroid cancer, basal cell carcinoma, Paget's disease or skin cancer.
(16) An agent increasing drug efficacy of a pharmaceutical composition for treatment and/or prevention of cancer comprising an antibody or a fragment thereof having an immunological reactivity with CAPRIN-1 protein as an active ingredient, wherein the agent comprises an immune checkpoint inhibitor as an active ingredient.
(17) An agent increasing drug efficacy of a pharmaceutical composition for treatment and/or prevention of cancer comprising an immune checkpoint inhibitor as an active ingredient, wherein the agent comprises an antibody or a fragment thereof having an immunological reactivity with CAPRIN-1 protein as an active ingredient.
(18) A method for treating and/or preventing cancer, comprising administering an antibody or a fragment thereof having an immunological reactivity with CAPRIN-1 protein, and an immune checkpoint inhibitor together or separately to a subject.

The present specification encompasses the contents disclosed in Japanese Patent Application No. 2020-043022 on which the priority of the present application is based.

### Advantageous Effects of Invention

The combination of an antibody against CAPRI-1 protein, or a fragment thereof, and an immune checkpoint inhibitor according to the present invention exerts a stronger antitumor effect than that of the antibody against CAPRIN-1 protein alone or an existing chemotherapeutic. The combination of an antibody against CAPRIN-1 protein, or a fragment thereof, and a drug comprising an immune checkpoint inhibitor according to the present invention exhibits a stronger antitumor effect than that of existing anticancer agent therapy or treatment with the antibody against CAPRIN-1 protein alone. Thus, the combination of the antibody against CAPRIN-1 protein, or the fragment thereof, and an immune checkpoint inhibitor is effective for treatment or prevention of cancer.

### Description of Embodiments

The antitumor activity of the combination of an antibody against CAPRIN-1 protein or a fragment thereof (hereinafter, referred to as an "anti-CAPRIN-1 antibody"), and an immune checkpoint inhibitor used in the present invention can be evaluated by examining *in vivo* the inhibition of tumor growth in a tumor-bearing animal as mentioned later.

The term "combination" described herein refers to simultaneous administration or administration in a predetermined interval of the anti-CAPRIN-1 antibody and an immune checkpoint inhibitor as independent active ingredients to the same organism. The interval may be simultaneous administration or may be 30 minutes later, 1 hour later, 3 hours later, 6 hours later, 12 hours later, 1 day later, 3 days later, 5 days later, 7 days later, 2 weeks later, 3 weeks later, or 4 weeks later. Combination of the anti-CAPRIN-1 antibody or a drug comprising an immune checkpoint inhibitor may be administered when another active ingredient exhibits its activity *in vivo.* The anti-CAPRIN-1 antibody may be administered first, or the drug comprising an immune checkpoint inhibitor may be administered first.

The term "comprising together or separately in combination" described herein refers to comprising a plurality of drugs in a form that allows the drugs to be administered simultaneously or separately to a patient. The form may be, for example, the form of a so-called mixed formulation in which a plurality of drugs is mixed, or may be the form of a so-called kit formulation comprising a plurality of drugs as individual formulations. The form also encompasses the form of a kit formulation comprising a plurality of drugs in any combination in two or more of formulations.

Such a kit formulation according to the present invention may be, for example, a kit formulation comprising a formulation (or a pharmaceutical composition) comprising the anti-CAPRIN-1 antibody and a formulation (or a pharmaceutical composition) comprising an immune checkpoint inhibitor. The kit formulation according to the present invention may comprise, in addition to the anti-CAPRIN-1 antibody and the immune checkpoint inhibitor, other drugs (other known antitumor agents or other immune checkpoint inhibitors) mentioned below.

The anti-CAPRIN-1 antibody according to the present invention may be a monoclonal antibody or a polyclonal antibody and is preferably a monoclonal antibody. The antibody of the present invention may be any type of antibody, as long as it can exhibit antitumor activity. The antibody is a recombinant antibody, a human antibody, a humanized antibody, a chimeric antibody, or a non-human animal antibody.

Subjects in need of treatment and/or prevention of cancer according to the present invention are mammals such as human, pet animals, livestock animals, or sport animals. The preferred subject is a human.

Medicaments comprising an anti-CAPRIN-1 antibody and an immune checkpoint inhibitor as active ingredients, and methods for treating and/or preventing cancer, related to the present invention, will be explained below.

### <Anti-CAPRIN-1 antibody>

Among CAPRIN-1 proteins having an amino acid sequence shown in any one of the even numbered SEQ ID NOs: 2 to 30, which are specific examples of antigens having an immunological reactivity with the anti-CAPRIN-1 antibody used in the present invention, the amino acid sequences shown in SEQ ID NOs: 6, 8, 10, 12 and 14 are amino acid sequences of canine CAPRIN-1 proteins; the amino acid sequences shown in SEQ ID NOs: 2 and 4 are amino acid sequences of human CAPRIN-1 proteins; the amino acid sequence shown in SEQ ID NO: 16 is an amino acid sequence of a bovine CAPRIN-1 protein; the amino acid sequence shown in SEQ ID NO: 18 is an amino acid sequence of a horse CAPRIN-1 protein; the amino acid sequences shown in SEQ ID NOs: 20, 22, 24 and 28 are amino acid sequences of mouse CAPRIN-1 proteins; and the amino acid sequence shown in SEQ ID NO: 30 is an amino acid sequence of a chicken CAPRIN-1 protein.

The anti-CAPRIN-1 antibody used in the present invention may have an immunological reactivity with a CAPRIN-1 protein variant having 80% or more, preferably 90% or more, more preferably 95% or more, and further preferably 99% or more sequence identity to the amino acid sequence shown in any one of the even numbered SEQ ID NOs: 2 to 30. The term "% sequence identity" as used herein means a percentage (%) of the number of identical amino acids (or nucleotides) to the total number of amino acids (or nucleotides) in the case that two sequences are aligned such that maximum similarity can be achieved with or without introduction of gaps.

In the present invention, the anti-CAPRIN-1 antibody refers to an antibody or a fragment (antigen binding fragment) thereof having an immunological reactivity with a full-length CAPRIN-1 protein or a fragment thereof. The term "immunological reactivity" used herein indicates the characteristics of an antibody specifically binding *in vivo* or *in vitro* to a CAPRIN-1 protein or a partial polypeptide thereof.

The anti-CAPRIN-1 antibody used in the present invention may be a monoclonal antibody or a polyclonal antibody.

Polyclonal antibodies having an immunological reactivity with a full-length CAPRIN-1 protein or a fragment thereof (anti-CAPRIN-1 polyclonal antibodies) can be obtained, for example, in a manner described below. Mice, human antibody-producing mice, rats, rabbits, chickens, or the like are immunized using a naturally occurring CAPRIN-1 protein or a protein fused with GST or the like, or a partial peptide thereof, followed by obtainment of serum, and then by purification from the obtained serum via ammonium sulfate precipitation, protein A, protein G, DEAE ion-exchange columns, affinity columns to which a CAPRIN-1 protein or a partial peptide is coupled, or the like.

Nucleotide sequences and amino acid sequences of CAPRIN-1 and homologs thereof can be obtained by, for example, accessing the website of GenBank (NCBI, USA) and using the BLAST or FASTA algorithm (Karlin and Altschul, Proc. Natl. Acad. Sci. USA, 90: 5873-5877, 1993; and Altschul et al., Nucleic Acids Res. 25: 3389-3402, 1997). Methods for producing CAPRIN-1 protein can be obtained with reference to WO2014/012479 or may employ cells or the like expressing CAPRIN-1 protein.

Monoclonal antibodies having an immunological reactivity with a full-length CAPRIN-1 protein or a fragment thereof (anti-CAPRIN-1 monoclonal antibodies) can be obtained, for example, in a manner described below. Breast cancer cells SK-BR-3 expressing CAPRIN-1, a full-length CAPRIN-1 protein or a fragment thereof, or the like is administered to mice for immunization. Splenocytes separated from the mice are fused with myeloma cells. Clones capable of producing anti-CAPRIN-1 monoclonal antibodies can be selected from the obtained fusion cells (hybridomas) to obtain these antibodies. The antibodies produced from the selected hybridomas can be obtained in the same way as the aforementioned method for purifying polyclonal antibodies.

The antibody used in the present invention includes human antibodies, humanized antibodies, chimeric antibodies, and non-human animal antibodies.

For human antibodies, human lymphocytes infected with EB virus are sensitized with a protein, protein-expressing cells, or a lysate thereof. The sensitized lymphocytes are fused with human-derived myeloma cells such as U266 cells. Antibodies having an immunological reactivity with a full-length CAPRIN-1 protein or a fragment thereof can be obtained from the obtained fusion cells.

A humanized antibody is a modified antibody, and it is sometimes referred to as a reshaped human antibody. It is known that a humanized antibody is constructed by transplanting complementarity determining regions of an immunized animal-derived antibody into complementarity determining regions of a human antibody. In addition, a general gene recombinant technique therefor is well known. Specifically, a DNA sequence designed in a manner that allows complementarity determining regions of mouse or rabbit antibody to be ligated to human antibody framework regions is synthesized by the PCR method using several oligonucleotides prepared in such a manner that the oligonucleotides have portions overlapping each other at one end of each thereof. A humanized antibody can be obtained by ligating the above obtained DNA to DNA encoding a human antibody constant region, incorporating the resultant into an expression vector, and introducing the vector into a host for antibody production (see EP-A-239400 and WO96/02576). Framework regions of human antibody ligated to each other via complementarity determining regions are selected on the assumption that complementarity determining regions can form an effective antigen binding site. If necessary, amino acids in framework regions of an antibody variable region may be substituted in such a manner that complementarity determining regions in a reshaped human antibody form an appropriate antigen binding site (Sato K. et al., Cancer Research 1993, 53: 851-856). In addition, the framework regions may be substituted with framework regions from a different human antibody (see WO99/51743).

In general, antibodies are heteromultimeric glycoproteins each comprising at least two heavy chains and two light chains. Antibodies each comprise two identical light chains and two identical heavy chains. Each heavy chain has a heavy-chain variable region at one end thereof, to which some constant regions are bound in series. Each light chain has a light-chain variable region at one end thereof to which some constant regions are bound in series. Variable regions have a specific variable region, which is called complementarity determining region (CDR) and imparts binding specificity to an antibody. A relatively conserved portion in a variable region is called a framework region (FR). A complete heavy-chain or light-chain variable region comprises 4 FRs connected to each other via 3 CDRs (CDR1 to CDR3).

Sequences of human-derived heavy-chain and light-chain constant regions and variable regions can be obtained from, for example, NCBI (USA; GenBank, UniGene, etc.). For example, for a human IgG1 heavy-chain constant region, see registration No. J00228; for a human IgG2 heavy-chain constant region, see registration No. J00230; for a human light chain κ constant region, see sequences such as registration Nos. V00557, X64135, and X64133; and for a human light chain λ constant region, see sequences such as registration Nos. X64132 and X64134.

A chimeric antibody is an antibody produced by combining sequences from different animals. An example thereof is an antibody consisting of mouse antibody heavy-chain and light-chain variable regions and constant regions of human antibody heavy-chain and light-chain variable regions. Such a chimeric antibody can be produced by a known method. For example, it can be obtained by ligating DNA encoding an antibody V region to DNA encoding a human antibody C region, incorporating the resultant into an expression vector, and introducing the vector into a host for antibody production.

Non-human animal antibodies are obtained by immunizing animals with sensitizing antigens according to a known method or by intraperitoneally, intracutaneously, or subcutaneously injecting sensitizing antigens into animals such as mice as a general method. For injecting sensitizing antigens, an appropriate amount of various adjuvants including CFA (complete Freund's adjuvant) is mixed therewith and the mixture is administered to animals several times. After immunization of animals and confirmation of an anti-CAPRIN-1 antibody contained in serum, the serum is obtained and a non-human animal antibody can be obtained by purification via ammonium sulfate precipitation, protein A, protein G, DEAE ion-exchange columns, affinity columns to which a CAPRIN-1 protein or a partial peptide is coupled, or the like, as mentioned above. In the case of obtaining monoclonal antibodies from non-human animals, a monoclonal antibody is obtained by collecting immune cells from the immunized animals and subjecting them to cell fusion with myeloma cells. The cell fusion of immune cells with myeloma cells can be carried out according to a known method (see Kohler, G. and Milstein, C. Methods Enzymol. (1981) 73, 3-46).

The antibody used in the present invention can also be obtained as a gene recombinant antibody produced by cloning an antibody gene from a hybridoma, incorporating the clone into an adequate vector, introducing the vector into a host, and producing the antibody by using a gene recombinant technique (see Carl, A.K. Borrebaeck, James, W. Larrick, THERAPEUTIC MONOCLONAL ANTIBODIES, Published in the United Kingdom by MACMILLAN PUBLISHERS LTD, 1990).

Amino acids in a variable region (e.g., FR) or a constant region in the anti-CAPRIN-1 antibody used in the present invention may be substituted with different amino acids. The amino acid substitution is a substitution of 1 or several, for example, less than 15, less than 10, not more than 8, not more than 6, not more than 5, not more than 4, not more than 3, or not more than 2 amino acids, preferably 1 to 9 amino acids. A substituted antibody should have characteristics of specifically binding to the antigen and binding affinity for the antigen equivalent to or higher than those of an unsubstituted antibody and should be an antibody that causes no rejection when applied to humans. The amino acid substitution is preferably a conservative amino acid substitution, which is a substitution between amino acids having similar characteristics in terms of charge, side chains, polarity, aromaticity, and the like. For example, characteristically similar amino acids can be classified into the following types: basic amino acids (arginine, lysine, and histidine); acidic amino acids (aspartic acid and glutamic acid); uncharged polar amino acids (glycine, asparagine, glutamine, serine, threonine, cysteine, and tyrosine); nonpolar amino acids (leucine, isoleucine, alanine, valine, proline, phenylalanine, tryptophan, and methionine); branched-chain amino acids (threonine, valine, isoleucine); and aromatic amino acids (phenylalanine, tyrosine, tryptophan, and histidine).

The anti-CAPRIN-1 antibody used in the present invention is expected to have a stronger antitumor effect when having higher binding affinity for CAPRIN-1 protein on the cancer cell surface. Association constant (affinity constant) Ka (kon/koff) is preferably at least 10⁷ M⁻¹, at least 10⁸ M⁻¹, at least 5 × 10⁸ M⁻¹, at least 10⁹ M⁻¹, at least 5 × 10⁹ M⁻¹, at least 10¹⁰ M⁻¹, at least 5 × 10¹⁰ M⁻¹, at least 10¹¹ M⁻¹, at least 5 × 10¹¹ M⁻¹, at least 10¹² M⁻¹, or at least 10¹³ M⁻¹.

The anti-CAPRIN-1 antibody used in the present invention may be chemically modified. Examples of such an antibody modifier can include antibodies bound to various molecules such as polyethylene glycol (PEG) and antitumor compounds (for example, antitumor agents listed below). Regarding antibody modifiers of the present invention, substances that bind to an antibody are not limited. Such an antibody modifier can be obtained by chemically modifying an obtained antibody. Methods of such modification have been already established in the field related to the present invention.

The binding strength of the anti-CAPRIN-1 antibody used in the present invention against effector cells can be improved by substituting 1, 2 or several amino acids in the heavy-chain constant region of the antibody or by removing fucose bound to N-acetylglucosamine in a N-glycoside-linked sugar chain bound to the heavy-chain constant region. The anti-CAPRIN-1 antibody described above may have the amino acid substitution alone or may be a composition with an antibody bound to fucose.

Antibodies in which 1, 2 or several amino acids in the heavy-chain constant region have been substituted can be produced with reference to, for example, WO2004/063351, WO2011/120135, U.S. Patent No. 8388955, WO2011/005481, U.S. Patent No. 6737056, and WO2005/063351.

Antibodies in which fucose bound to N-acetylglucosamine in a N-glycoside-linked sugar chain in the heavy-chain constant region has been removed, or producing cells thereof can be produced with reference to U.S. Patent No. 6602684, EP Patent No. 1914244, and U.S. Patent No. 7579170. Compositions of antibodies in which fucose bound to N-acetylglucosamine in a N-glycoside-linked sugar chain bound to the heavy-chain constant region has been removed, with antibodies bound to fucose, or producing cells thereof can be produced with reference to, for example, U.S. Patent No. 8642292.

The anti-CAPRIN-1 polyclonal antibody and the anti-CAPRIN-1 monoclonal antibody used in the present invention, methods for producing or purifying antibodies and methods for producing a CAPRIN-1 protein or partial polypeptide thereof used in immunization can be obtained with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212.

Specific examples of the anti-CAPRIN-1 antibody according to the present invention include anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212 mentioned above. Preferred examples of the anti-CAPRIN-1 antibody include the following.

An antibody or a fragment thereof having an immunological reactivity with a partial polypeptide of a CAPRIN-1 protein having the amino acid sequence shown in SEQ ID NO: 2 or SEQ ID NO: 4 or an amino acid sequence having 80% or more (preferably 85% or more, more preferably 90% or more, further preferably 95% or more, and still further preferably 99% or more) sequence identity to the amino acid sequence.

An antibody or a fragment thereof having an immunological reactivity with a partial polypeptide of CAPRIN-1 protein, the partial polypeptide having the amino acid sequence shown in SEQ ID NO: 31 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with a CAPRIN-1 protein, an antibody or a fragment thereof comprising a heavy-chain variable region comprising complementarity determining regions of SEQ ID NOs: 140, 141 and 142 (CDR1, CDR2 and CDR3, respectively) and a light-chain variable region comprising complementarity determining regions of SEQ ID NOs: 143, 144 and 145 (CDR1, CDR2 and CDR3, respectively), and having an immunological reactivity with a CAPRIN-1 protein, or an antibody or a fragment thereof comprising a heavy-chain variable region comprising complementarity determining regions of SEQ ID NOs: 164, 165 and 166 (CDR1, CDR2 and CDR3, respectively) and a light-chain variable region comprising complementarity determining regions of SEQ ID NOs: 167, 168 and 169 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 39 and a light-chain variable region comprising the amino acid sequence of SEQ ID NO: 43, an antibody or a fragment thereof comprising a heavy-chain variable region comprising the amino acid sequence of SEQ ID NO: 70 and a light-chain variable region comprising the amino acid sequence of SEQ ID NO: 71, or an antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof having an immunological reactivity with a partial polypeptide of CAPRIN-1 protein, the partial polypeptide having the amino acid sequence shown in SEQ ID NO: 33 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 63 and a light-chain variable region comprising the amino acid sequence of SEQ ID NO: 67.

An antibody or a fragment thereof having an immunological reactivity with a partial polypeptide of CAPRIN-1 protein, the partial polypeptide having the amino acid sequence shown in SEQ ID NO: 32 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 55 and a light-chain variable region comprising the amino acid sequence of SEQ ID NO: 59.

An antibody or a fragment thereof having an immunological reactivity with a partial polypeptide of CAPRIN-1 protein, the partial polypeptide having the amino acid sequence shown in SEQ ID NO: 34 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with a CAPRIN-1 protein, or an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 80 and a light-chain variable region comprising the amino acid sequence of SEQ ID NO: 81, or an antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof having an immunological reactivity with a partial polypeptide of CAPRIN-1 protein, the partial polypeptide having the amino acid sequence shown in SEQ ID NO: 35 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with a CAPRIN-1 protein, or an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 84 and a light-chain variable region comprising the amino acid sequence of SEQ ID NO: 85, or an antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with a CAPRIN-1 protein, and preferably an antibody or a fragment thereof comprising a heavy-chain variable region comprising the amino acid sequence of SEQ ID NO: 47 and a light-chain variable region comprising the amino acid sequence of SEQ ID NO: 51.

An antibody or a fragment thereof having an immunological reactivity with a partial polypeptide of CAPRIN-1 protein, the partial polypeptide having the amino acid sequence shown in SEQ ID NO: 296 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof having an immunological reactivity with a partial polypeptide of CAPRIN-1 protein, the partial polypeptide having the amino acid sequence shown in SEQ ID NO: 297 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof having an immunological reactivity with a partial polypeptide of CAPRIN-1 protein, the partial polypeptide having the amino acid sequence shown in SEQ ID NO: 298 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof having an immunological reactivity with a partial polypeptide of CAPRIN-1 protein, the partial polypeptide having the amino acid sequence shown in SEQ ID NO: 299 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 300 and a light-chain variable region comprising the amino acid sequence of SEQ ID NO: 304.

An antibody or a fragment thereof having an immunological reactivity with a partial polypeptide of CAPRIN-1 protein, the partial polypeptide having the amino acid sequence shown in SEQ ID NO: 308 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof having an immunological reactivity with a partial polypeptide of CAPRIN-1 protein, the partial polypeptide having the amino acid sequence shown in SEQ ID NO: 309 or an amino acid sequence having 80% or more (preferably 85% or more, more preferably 90% or more, and further preferably 95% or more) sequence identity to the amino acid sequence, preferably an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with a CAPRIN-1 protein, and more preferably an antibody or a fragment thereof comprising a heavy-chain variable region comprising the amino acid sequence of SEQ ID NO: 68 and a light-chain variable region comprising the amino acid sequence of SEQ ID NO: 69.

In addition, the following anti-CAPRIN-1 antibodies are preferably used.

An antibody or a fragment thereof comprising a heavy-chain variable region comprising the amino acid sequence of SEQ ID NO: 68 and a light-chain variable region comprising the amino acid sequence of SEQ ID NO: 69.

An antibody or a fragment thereof comprising a heavy-chain variable region comprising the amino acid sequence of SEQ ID NO: 70 and a light-chain variable region comprising the amino acid sequence of SEQ ID NO: 71.

An antibody or a fragment thereof comprising a heavy-chain variable region comprising the amino acid sequence of SEQ ID NO: 72 and a light-chain variable region comprising the amino acid sequence of SEQ ID NO: 73.

An antibody or a fragment thereof comprising a heavy-chain variable region comprising the amino acid sequence of SEQ ID NO: 74 and a light-chain variable region comprising the amino acid sequence of SEQ ID NO: 75.

An antibody or a fragment thereof comprising a heavy-chain variable region comprising the amino acid sequence of SEQ ID NO: 76 and a light-chain variable region comprising the amino acid sequence of SEQ ID NO: 77.

An antibody or a fragment thereof comprising a heavy-chain variable region comprising the amino acid sequence of SEQ ID NO: 78 and a light-chain variable region comprising the amino acid sequence of SEQ ID NO: 79.

An antibody or a fragment thereof comprising a heavy-chain variable region comprising the amino acid sequence of SEQ ID NO: 80 and a light-chain variable region comprising the amino acid sequence of SEQ ID NO: 81.

An antibody or a fragment thereof comprising a heavy-chain variable region comprising the amino acid sequence of SEQ ID NO: 82 and a light-chain variable region comprising the amino acid sequence of SEQ ID NO: 83.

An antibody or a fragment thereof comprising a heavy-chain variable region comprising the amino acid sequence of SEQ ID NO: 84 and a light-chain variable region comprising the amino acid sequence of SEQ ID NO: 85.

An antibody or a fragment thereof comprising a heavy-chain variable region comprising the amino acid sequence of SEQ ID NO: 86 and a light-chain variable region comprising the amino acid sequence of SEQ ID NO: 87.

An antibody or a fragment thereof comprising a heavy-chain variable region comprising the amino acid sequence of SEQ ID NO: 88 and a light-chain variable region comprising the amino acid sequence of SEQ ID NO: 89.

An antibody or a fragment thereof comprising a heavy-chain variable region comprising the amino acid sequence of SEQ ID NO: 90 and a light-chain variable region comprising the amino acid sequence of SEQ ID NO: 91.

An antibody or a fragment thereof comprising a heavy-chain variable region comprising the amino acid sequence of SEQ ID NO: 92 and a light-chain variable region comprising the amino acid sequence of SEQ ID NO: 93.

An antibody or a fragment thereof comprising a heavy-chain variable region comprising the amino acid sequence of SEQ ID NO: 94 and a light-chain variable region comprising the amino acid sequence of SEQ ID NO: 95.

An antibody or a fragment thereof comprising a heavy-chain variable region comprising the amino acid sequence of SEQ ID NO: 96 and a light-chain variable region comprising the amino acid sequence of SEQ ID NO: 97.

An antibody or a fragment thereof comprising a heavy-chain variable region comprising the amino acid sequence of SEQ ID NO: 98 and a light-chain variable region comprising the amino acid sequence of SEQ ID NO: 99.

An antibody or a fragment thereof comprising a heavy-chain variable region comprising the amino acid sequence of SEQ ID NO: 100 and a light-chain variable region comprising the amino acid sequence of SEQ ID NO: 101.

An antibody or a fragment thereof comprising a heavy-chain variable region comprising the amino acid sequence of SEQ ID NO: 102 and a light-chain variable region comprising the amino acid sequence of SEQ ID NO: 103.

An antibody or a fragment thereof comprising a heavy-chain variable region comprising the amino acid sequence of SEQ ID NO: 104 and a light-chain variable region comprising the amino acid sequence of SEQ ID NO: 105.

An antibody or a fragment thereof comprising a heavy-chain variable region comprising the amino acid sequence of SEQ ID NO: 106 and a light-chain variable region comprising the amino acid sequence of SEQ ID NO: 107.

An antibody or a fragment thereof comprising a heavy-chain variable region comprising the amino acid sequence of SEQ ID NO: 108 and a light-chain variable region comprising the amino acid sequence of SEQ ID NO: 109.

An antibody or a fragment thereof comprising a heavy-chain variable region comprising the amino acid sequence of SEQ ID NO: 110 and a light-chain variable region comprising the amino acid sequence of SEQ ID NO: 111.

An antibody or a fragment thereof comprising a heavy-chain variable region comprising the amino acid sequence of SEQ ID NO: 112 and a light-chain variable region comprising the amino acid sequence of SEQ ID NO: 113.

An antibody or a fragment thereof comprising a heavy-chain variable region comprising the amino acid sequence of SEQ ID NO: 114 and a light-chain variable region comprising the amino acid sequence of SEQ ID NO: 115.

An antibody or a fragment thereof comprising a heavy-chain variable region comprising the amino acid sequence of SEQ ID NO: 116 and a light-chain variable region comprising the amino acid sequence of SEQ ID NO: 117.

An antibody or a fragment thereof comprising a heavy-chain variable region comprising the amino acid sequence of SEQ ID NO: 118 and a light-chain variable region comprising the amino acid sequence of SEQ ID NO: 119.

An antibody or a fragment thereof comprising a heavy-chain variable region comprising the amino acid sequence of SEQ ID NO: 120 and a light-chain variable region comprising the amino acid sequence of SEQ ID NO: 121.

An antibody or a fragment thereof comprising a heavy-chain variable region comprising the amino acid sequence of SEQ ID NO: 122 and a light-chain variable region comprising the amino acid sequence of SEQ ID NO: 123.

An antibody or a fragment thereof comprising a heavy-chain variable region comprising the amino acid sequence of SEQ ID NO: 124 and a light-chain variable region comprising the amino acid sequence of SEQ ID NO: 125.

An antibody or a fragment thereof comprising a heavy-chain variable region comprising the amino acid sequence of SEQ ID NO: 126 and a light-chain variable region comprising the amino acid sequence of SEQ ID NO: 127.

An antibody or a fragment thereof comprising a heavy-chain variable region comprising the amino acid sequence of SEQ ID NO: 128 and a light-chain variable region comprising the amino acid sequence of SEQ ID NO: 129.

An antibody or a fragment thereof comprising a heavy-chain variable region comprising the amino acid sequence of SEQ ID NO: 130 and a light-chain variable region comprising the amino acid sequence of SEQ ID NO: 131.

An antibody or a fragment thereof comprising a heavy-chain variable region comprising the amino acid sequence of SEQ ID NO: 132 and a light-chain variable region comprising the amino acid sequence of SEQ ID NO: 133.

An antibody or a fragment thereof comprising a heavy-chain variable region comprising the amino acid sequence of SEQ ID NO: 300 and a light-chain variable region comprising the amino acid sequence of SEQ ID NO: 304.

In Examples mentioned later, a polyclonal antibody or a monoclonal antibody against full-length CAPRIN-1 protein or a polypeptide of a portion of an extracellular region expressed on the cell membrane surface of cancer cells was confirmed to exhibit a reactivity with the cell membrane surfaces of a plurality of human cancer cells. Also, results indicating that human cancer patients responded thereto were obtained, and a marked antitumor effect was obtained by which tumor completely disappeared at some cancer sites.

### <Immune checkpoint>

The immune checkpoint as used herein refers to a function that allows immune cells and cancer cells to recognize each other's antigens *in vivo* so that stimulate them or inhibit stimuli. Specifically, "PD-L1", "PD-L2", "CD80" or "CD86" is expressed as a molecule for activating the immune checkpoint on cell membrane surface of antigen-presenting cells such as dendritic cells, macrophages, or cancer cells. On the other hand, a molecule "PD-1 (programmed death-1)", "CD28", or "CTLA-4 (cytotoxic T-lymphocyte associated antigen-4)" which interacts with "PD-L1", "PD-L2", "CD80" or "CD86" is expressed on immune cells such as T lymphocytes. These molecules are so-called immune checkpoint molecules.

The interaction used herein is action by which molecules to be paired with each other stimulate or inhibit the functions of each other's cells through association or dissociation. For example, *in vivo* cancer cells often express "PD-L1" or "PD-L2". When "PD-1" expressed on T cells binds to this molecule, the ability of the T cells to produce cytokines is reduced so that signals that suppress the activity of the T cells are transduced into the cells. Cancer cells often express the PD-L1 molecule and escape the surveillance functions or attack functions *of in vivo* T cells through the interaction.

"CTLA-4", an immune checkpoint molecule, is expressed on activated T cells or immunosuppressive T cells (regulatory T cells). Th molecule "CD80" or "CD86" with which CTLA-4 interacts is expressed on antigen-presenting cells, and CTLA-4 binds to CD80 or CD86. Since CTLA-4 often binds more strongly than the molecule CD28 which also interacts with CD80 or CD86, maturation of the antigen-presenting cells is suppressed, resulting in suppressed activation of the T cells.

PD-L1 expression is observed on antigen-presenting cells or various cancer cells such as lung cancer, colon cancer, or malignant melanoma. PD-L2 is expressed on activated antigen-presenting cells, macrophages, or cancer cells.

### <Immune checkpoint inhibitor>

A drug that cancels an *in vivo* suppressed state by inhibiting the functions of the immune checkpoint is called "immune checkpoint inhibitor". The immune checkpoint inhibitor is, for example, preferably an anti-PD-L1 antibody as an inhibitor for PD-L1, preferably an anti-PD-1 antibody as an inhibitor for PD-1, and preferably an anti-CTLA-4 antibody as an inhibitor for CTLA-4.

The anti-PD-1 antibody is preferably nivolumab, pembrolizumab, spartalizumab, and/or cemiplimab.

The anti-PD-L1 antibody is preferably avelumab, atezolizumab, and/or durvalumab.

The anti-CTLA-4 antibody is preferably ipilimumab and/or tremelimumab.

Other examples of an antibody serving as the immune checkpoint inhibitor include anti-PD-1/CTLA-4 bispecific antibodies (e.g., MEDI5752).

### <Other drugs>

The anti-CAPRIN-1 antibody and the drug comprising an immune checkpoint inhibitor serving as active ingredients in the medicament of the present invention may be combined with one or more other immune checkpoint inhibitors or may be combined with an antitumor agent known in literatures, etc. Specific examples of known antitumor agents include, but are not particularly limited to, padoxorubicin, daunorubicin, cyclophosphamide, methotrexate, thiotepa, busulfan, improsulfan, piposulfan, benzodopa, carboquone, meturedopa, uredopa, altretamine, triethylenemelamine, triethylenephosphoramide, triethilenethiophosphoramide, trimethylolomelamine, bullatacin, bullatacinone, camptothecin, bryostatin, callystatin, cryptophycin 1, cryptophycin 8, dolastatin, duocarmycin, eleutherobin, pancratistatin, sarcodictyin, spongistatin, chlorambucil, chloRNAphazine, cholophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, calicheamicin, dynemicin, clodronate, esperamicin, aclacinomycin, actinomycin, authramycin, azaserine, bleomycin, cactinomycin, carabicin, carminomycin, carzinophilin, chromomycin, dactinomycin, detorbicin, 6-diazo-5-oxo-L-norleucine, adriamycin, epirubicin, esorubicin, idarubicin, marcellomycin, mitomycin C, mycophenolic acid, nogalamycin, olivomycin, peplomycin, potfiromycin, puromycin, quelamycin, rodorubicin, streptonigrin, streptozocin, tubercidin, ubenimex, zinostatin, zorubicin, denopterin, pteropterin, trimetrexate, fludarabine, 6-mercaptopurine, thiamiprine, thioguanine, ancitabine, azacitidine, 6-azauridine, carmofur, dideoxyuridine, enocitabine, floxuridine, androgens such as calusterone, dromostanolone propionate, epitiostanol, mepitiostane, testolactone, aminoglutethimide, mitotane, trilostane, frolinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, epothilone, etoglucid, lentinan, lonidamine, maytansine, ansamitocine, mitoguazone, mitoxantrone, mopidanmol, nitraerine, pentostatin, phenamet, pirarubicin, losoxantrone, podophyllinic acid, 2-ethylhydrazide, procarbazine, razoxane, rhizoxin, schizophyllan, spirogermanium, tenuazonic acid, triaziquone, roridine A, anguidine, urethane, vindesine, dacarbazine, mannomustine, mitobronitol, mitolactol, pipobroman, gacytosine, dokicetaxel, chlorambucil, 6-thioguanine, mercaptopurine, oxaliplatin, vinblastine, etoposide, ifosfamide, mitoxantrone, vincristine, vinorelbine, novantrone, teniposide, edatrexate, daunomycin, aminopterin, xeloda, ibandronate, irinotecan, topoisomerase inhibitor, difluoromethylornithine (DMFO), retinoic acid, and pharmacologically acceptable (known) salts or (known) derivatives thereof.

### <Antitumor effect of present invention>

A combination of the anti-CAPRIN-1 antibody and an immune checkpoint inhibitor of the present invention has cytotoxic activity *in vivo.* Accordingly, the antitumor effect of the present invention can be determined by examining cytotoxic activity against cancer. The cytotoxic activity can be evaluated by administering a drug comprising the anti-CAPRIN-1 antibody and an immune checkpoint inhibitor to an organism having cancer, measuring the size of a tumor after the administration, and examining the size of the cancer over time. Also, the antitumor effect of the present invention can be evaluated by examining a survival rate. Alternatively, the antitumor effect of the present invention may be evaluated by examining the ability to produce cytokines or chemokines. The antitumor effect of the combination of the anti-CAPRIN-1 antibody and an immune checkpoint inhibitor, etc. according to the present invention, or the antitumor effect of the combination of the anti-CAPRIN-1 antibody, a drug comprising an immune checkpoint inhibitor, and one or more additional drugs according to the present invention can be further determined by examining prevention of cancer, prevention of metastasis or prevention of recurrence.

The anti-CAPRIN-1 antibody used in the present invention can be expected to have a stronger antitumor effect when having higher binding affinity for CAPRIN-1 protein on the cancer cell surface. Association constant (affinity constant) Ka (kon/koff) is preferably at least 10⁷ M⁻¹, at least 10⁸ M⁻¹, at least 5 × 10⁸ M⁻¹, at least 10⁹ M⁻¹, at least 5 × 10⁹ M⁻¹, at least 10¹⁰ M⁻¹, at least 5 × 10¹⁰ M⁻¹, at least 10¹¹ M⁻¹, at least 5 × 10¹¹ M⁻¹, at least 10¹² M⁻¹, or at least 10¹³ M⁻¹.

An ability of an anti-CAPRIN-1 antibody used in the present invention to bind to CAPRIN-1 can be specified via binding assays using, for example, ELISA, Western blot, immunofluorescence, or flowcytometry analysis.

Administration of a combination of an anti-CAPRIN-1 antibody, an angiogenesis inhibitor, and carboplatin according to the present invention to an organism having cancer increases an antitumor effect as compared with an anti-CAPRIN-1 antibody alone, as mentioned above. The rate of increase is preferably 30% or more, more preferably 40% or more, further preferably 50% or more, still further preferably 55% or more, even further preferably 60% or more, even further preferably 65% or more, and most preferably 70% or more. The rate of increase in antitumor effect by administration of a combination of an anti-CAPRIN-1 antibody, an angiogenesis inhibitor, and carboplatin according to the present invention with respect to administration of the anti-CAPRIN-1 antibody alone can be calculated by administering their respective effective amounts to cancer-bearing mice under the same conditions, and comparing tumor volumes on 7 days or later after the start of administration.

### <Medicament for treatment and/or prevention of cancer>

A medicament of the present invention is aimed at treating and/or preventing cancer. A cancer targeted by the medicament of the present invention is not particularly limited as long as it is cancer (cells) expressing CAPRIN-1 protein or cancer expressing an immune checkpoint molecule (e.g., PD-L1 or PD-L2).

The term "treatment" used herein refers to treatment of cancer based on an antitumor effect mentioned above. The term "prevention" used herein refers to not only prevention of development of cancer but prevention of metastasis or recurrence of cancer.

Both the terms "tumor" and "cancer" used herein refer to malignant neoplasm, and thus they are used in an exchangeable manner.

A cancer patient that can be a target in the present invention is not particularly limited and is preferably a cancer patient with a previous history of cancer treatment with a medicament other than cancer treatment with a medicament comprising an antibody or a fragment thereof having an immunological reactivity with CAPRIN-1 protein, and an immune checkpoint inhibitor. The cancer patient also includes a patient treated with a chemotherapeutic, a molecular targeted drug, or hormone therapy in the past. Examples thereof include cancer patients who have undergone cancer treatment in accordance with "NCCN Clinical Practice Guidelines in Oncology", "ESMO Clinical Practice Guidelines" or "Clinical Practice Guideline". A cancer patient with a previous history of cancer treatment with an immune checkpoint inhibitor is preferred.

The patient is preferably a cancer patient who has not responded to cancer treatment with a medicament other than cancer treatment with a medicament comprising an anti-CAPRIN-1 antibody and an immune checkpoint inhibitor together or separately in combination, and more preferably a cancer patient who has not responded to cancer treatment with an immune checkpoint inhibitor.

The patient is preferably a cancer patient with cancer resistant to cancer treatment with a medicament other than cancer treatment with a medicament comprising an anti-CAPRIN-1 antibody and an immune checkpoint inhibitor together or separately in combination, and more preferably a cancer patient with cancer resistant to cancer treatment with an immune checkpoint inhibitor. The terms "having not responded to cancer treatment" and "resistant to cancer treatment" described herein are used to have the same meaning.

Cancer that can be a target in the present invention is any cancer as long as the cancer expresses CAPRIN-1 protein on the cell membrane surface. The cancer is preferably melanoma (including postoperative melanoma), lung cancer (including non-small cell lung cancer and small cell lung cancer), renal cell carcinoma, Hodgkin's lymphoma, head and neck cancer, gastric cancer, mesothelioma (including malignant pleural mesothelioma), colorectal cancer (e.g., MSI-high colorectal cancer), esophageal cancer, gastroesophageal junction cancer, hepatocellular carcinoma, glioblastoma, urothelial carcinoma, ovarian cancer, urinary bladder cancer, uterine cancer (including uterine cervical cancer and uterine body cancer), primary central nervous system lymphoma, primary testicular lymphoma, pancreatic cancer, biliary tract cancer, bile duct cancer, breast cancer, kidney cancer, colon cancer, brain tumor, prostate cancer, leukemia, lymphoma, liver cancer, sarcoma, fibrosarcoma, mastocytoma, adrenocortical carcinoma, Ewing's tumor, multiple myeloma, testicular cancer, thyroid cancer, basal cell carcinoma, Paget's disease or skin cancer. These cancers may be primary cancer, metastatic cancer, metastasized cancer or relapsed cancer, postoperative cancer, or unresectable cancer. Melanoma is often used to have the same meaning as malignant melanoma.

Other examples of the cancer that can be a target in the present invention include cancer resistant to known treatment methods. The resistant cancer is not particularly limited and can be cancer derived from a patient with any history of treatment. The resistant cancer is, for example, cancer that is derived from a patient with a history of treatment with an immune checkpoint inhibitor and has no response, has become resistant, has metastasized, or has relapsed after administration.

More specifically, examples of the cancer include, but are not limited to, for example, Bowen's disease, prickle cell carcinoma, extramammary Paget's disease, mycosis fungoides, Sezary's syndrome, cutaneous T/NK-cell lymphoma, T-cell leukemia or lymphoma having a lesion only in the skin, cutaneous B-cell lymphoma (indolent group), cutaneous T-cell lymphatic breast adenocarcinoma, composite type breast adenocarcinoma, malignant mammary mixed tumor, intraductal papillary adenocarcinoma, lung adenocarcinoma, squamous cell cancer, small cell cancer, large cell cancer, glioma that is a tumor of neuroepithelial tissue, glioblastoma, neuroblastoma, ependymoma, neuronal tumor, embryonal neuroectodermal tumor, schwannoma, neurofibroma, meningioma, chronic lymphocytic leukemia, lymphoma, gastrointestinal lymphoma, digestive lymphoma, small-cell-to-medium-cell lymphoma, cecal cancer, ascending colon cancer, descending colon cancer, transverse colon cancer, sigmoid colon cancer, rectal cancer, ovarian epithelial cancer, germ cell tumor, interstitial cell tumor, pancreatic duct cancer, invasive pancreatic duct cancer, adenocarcinoma of pancreatic cancer, acinar cell carcinoma, adenosquamous carcinoma, giant cell tumor, intraductal papillary mucinous neoplasm, mucinous adenocarcinoma, pancreatoblastoma, pancreatic islet cell tumor, Frants tumor, serous cystadenocarcinoma, solid pseudopapillary cancer, gastrinoma, glucagonoma, insulinoma, multiple endocrine neoplasia type-1 (Wermer syndrome), nonfunctional islet cell tumor, somatostatinoma, VIPoma, uterine cervical cancer, uterine body cancer, fibrosarcoma, osteosarcoma, joint sarcoma, Ewing sarcoma, Wilms tumor, hepatoblastoma, soft tissue sarcoma, acute leukemia, chronic leukemia, spinal cord tumor, malignant soft tissue tumor, tumors of teratoma group, head and neck cancer including hypopharynx cancer, oropharynx cancer, tongue cancer, nasopharyngeal cancer, oral cavity cancer, lip cancer, nasal and sinus cancer, and laryngeal cancer, cancer of the renal pelvis and ureter, urinary bladder cancer, urethra cancer, testicular tumor, malignant pleural mesothelioma, malignant bone tumor, uterine body cancer (postoperative chemotherapy, chemotherapy at the time of metastasis or relapse), and pediatric malignant solid tumor (rhabdomyosarcoma, neuroblastoma, hepatoblastoma, medulloblastoma, nephroblastoma, retinoblastoma, central nervous system germ cell tumor, and Ewing sarcoma family of tumors). The cancer also includes a palpable cancer, a subcutaneously existing cancer, an intracutaneously existing cancer, a superficial cancer, cancer existing in the dermis and cancer existing in a non-parenchymal organ, advanced cancer, which originate from the cancers described above. The cancer also includes a palpable cancer, a subcutaneously existing cancer, an intracutaneously existing cancer, a superficial cancer, cancer existing in the dermis and cancer existing in a non-parenchymal organ, which originate from the cancers described above and have metastasized and recurred.

A preferable subject (patient) that can be a target is a mammal and is, for example, a mammal including primates, pet animals, livestock animals, and sport animals. Humans, dogs and cats are particularly preferable.

A medicament of the present invention can be formulated by a method known to persons skilled in the art. For instance, the medicament of the present invention can be parenterally used in the form of a parenteral injection of: an aseptic solution in water or a pharmacologically acceptable non-water solution; or a suspension liquid. In the medicament of the present invention, an active ingredient (at least one of an anti-CAPRIN-1 antibody of the present invention and an immune checkpoint inhibitor) of each formulation or pharmaceutical composition may be combined with, for example, a pharmacologically acceptable carrier, medium, or additive, specifically, sterilized water, physiological saline, an isotonic solution, a buffering agent (buffer solution, etc.), plant oil, oily liquid, an antioxidant, a dissolution aid, an emulsifier, a suspension, a surfactant, a stabilizer, a fragrance, an excipient, or a binder in an appropriate manner, and preferably, may be formulated by mixing with them in a unit dosage form required for a generally acceptable pharmaceutical formulation. An amount of an active ingredient in a formulation is determined such that an appropriate dosage within an indicated range can be achieved.

An aseptic composition for injection can be prepared in accordance with general formulation practice using a vehicle such as distilled water for injection. An aqueous solution for injection includes, for example, saline or isotonic solutions comprising glucose and other adjuvants such as D-sorbitol, D-mannose, D-mannitol, and sodium chloride. Such solution may be used with an appropriate dissolution aid. Such dissolution aid includes, for example, alcohols such as ethanol and polyalcohol, such as propylene glycol, polyethylene glycol, or nonionic surfactants such as polysorbate 80(TM) and HCO-60. Oily liquid includes, for example, sesame oil or soybean oil. Such oily liquid may be used in combination with a dissolution aid such as benzyl benzoate or benzyl alcohol. In addition, it may be mixed with a buffering agent such as a phosphate buffer solution or a sodium acetate buffer solution, a soothing agent such as procaine hydrochloride, a stabilizer such as benzyl alcohol or phenol, or an antioxidant. In general, a formulated injection solution is introduced into an adequate ample.

The above pharmaceutical composition is orally or parenterally administered. Preferably, it is parenterally administered. Specifically, dosage forms include injectable agents, intranasally-administered agents, transpulmonarily-administered agents, and percutaneously-administered agents. For example, injectable agents can be systemically or locally administered via intravenous injection, intramuscular injection, intraperitoneal injection, subcutaneous injection, or intratumoral injection. The percutaneously-administered agents include, for example, agents called liniments and external medicines. The external medicines include, for example, solid agents, solutions, sprays, ointments, creams, and gels.

The administration method can be appropriately determined depending on age, weight, gender, and symptoms of a patient. A single dose of a pharmaceutical composition comprising at least one of an anti-CAPRIN-1 antibody and an immune checkpoint inhibitor can be selected within a range of, for example, 0.0001 mg to 1000 mg per kg of body weight as an amount of each active ingredient. Alternatively, the dose of each active ingredient can be selected within a range of, for example, 0.001 to 100000 mg per patient's body or 1 mg to 30 mg per kg of patient's body weight; however, it is not necessarily limited thereto. The dose and the administration method are changed depending on patient age, weight, gender, and symptoms. However, persons skilled in the art can appropriately select the dose and the method.

### <Administration method>

Treatment and/or prevention of cancer with a medicament for treatment and/or prevention of cancer of the present invention includes various modes, in addition to administration as a medicament mentioned above. For example, respective active ingredients in a medicament of the present invention can be administered simultaneously, concurrently, or individually in a staggered manner. As a specific example, active ingredients can be administered within a time interval up to approximately 3 weeks, i.e., the second active ingredient can be administered from immediately up to approximately 3 weeks after administration of the first active ingredient. These administrations may be carried out subsequently to a surgical procedure, or a surgical procedure may be carried out between the administrations of the first and second drugs. In addition, the medicament for treatment and/or prevention of cancer of the present invention may be administered according to a plurality of administration cycles. For example, in the case of carrying out simultaneous administration of respective active ingredients in a medicament for treatment and/or prevention of cancer of the present invention, a pharmaceutical composition comprising the active ingredients of the present invention (an anti-CAPRIN-1 antibody of the present invention and an immune checkpoint inhibitor) is administered for approximately 2 days to approximately 3 weeks as one cycle. Then, this treatment cycle may be repeated, if necessary, according to the judgment of a physician in charge. Likewise, in the case of scheduling a formulation in a staggered manner, respective administration periods of individual agents are adjusted so as to span the same period. The interval between cycles can vary from 0 to 2 months. Respective doses of the active ingredients in the medicament for treatment and/or prevention of cancer of the present invention can be set in the same way as in the respective doses of the active ingredients in the pharmaceutical composition described above.

### <Pharmaceutical kit>

A medicament for treatment and/or prevention of cancer of the present invention may be in the form of a pharmaceutical kit. The pharmaceutical kit is a package for using active ingredients in the form of separate pharmaceutical compositions (formulations) in a method for treating and/or preventing cancer. The package may comprise an instruction for administering each of the active ingredients. The respective active ingredients in the pharmaceutical compositions for treatment and/or prevention of cancer contained in the pharmaceutical kit can be in the form of pharmaceutical compositions each formulated as described above such that the active ingredients can be administered together or separately. Further, the pharmaceutical kit comprises active ingredients in amounts sufficient for one or more doses such that the active ingredients can be administered according to the administration method described above.

On the basis of the contents specifically described above, the present invention provides a method for treating and/or preventing cancer, comprising administering the medicament of the present invention, or the anti-CAPRIN-1 antibody of the present invention and an immune checkpoint inhibitor to a subject (patient). The present invention further provides, for example, a method for treating and/or preventing cancer, comprising administering the medicament of the present invention, etc. to a subject (patient) having cancer or suspected of having cancer. In the method of the present invention, in addition to the anti-CAPRIN-1 antibody of the present invention and the immune checkpoint inhibitor, other antitumor agents (known antitumor agents, etc.) may be administered to the subject (patient). In this embodiment, the anti-CAPRIN-1 antibody of the present invention (the antibody or the fragment thereof), the immune checkpoint inhibitor, and optionally, other antitumor agents contained in the medicament can be administered simultaneously or separately to the subject (patient).

### Examples

The present invention is hereafter described in detail with reference to the following examples, although the scope of the present invention is not limited thereto.

### (Example 1) Production of anti-CAPRIN-1 antibody

Anti-CAPRIN-1 antibodies having an immunological reactivity with CAPRIN-1 protein, used in the present invention were produced as described below for use.

### (Polyclonal antibody)

One (1) mg of a human CAPRIN-1 recombinant protein (SEQ ID NO: 2) produced according to Example 3 of WO2010/016526 was mixed with an incomplete Freund's adjuvant (IFA) solution in an amount equivalent to the recombinant protein. The mixture was subcutaneously administered to a rabbit 4 times every 2 weeks. Subsequently, blood was collected, so that an antiserum containing a polyclonal antibody was obtained. Furthermore, the antiserum was purified using a protein G carrier (GE Healthcare Bio-Sciences) and replaced with PBS(-) and then a polyclonal antibody against CAPRIN-1 protein (anti-CAPRIN-1 polyclonal antibody #1) was obtained.

### (Monoclonal antibody)

One hundred (100) µg of a human CAPRIN-1 recombinant protein produced according to Example 3 of WO2010/016526 was mixed with a MPL+TDM adjuvant (Sigma) in an amount equivalent to that of the recombinant protein. The mixture was used as an antigen solution per mouse. The antigen solution was administered intraperitoneally to 6-week-old Balb/c mice (Japan SLC Inc.) and then further administered 3 times and 24 times every week for completion of immunization. A spleen was removed on day 3 after the final immunization and then ground between two sterilized glass slides. Spleen cells were obtained by washing with PBS (-) (Nissui Pharmaceutical), centrifuging at 1500 rpm for 10 minutes, and removing supernatant, therein these were repeated 3 times. The obtained spleen cells were mixed with mouse myeloma cell SP2/0 (purchased from ATCC) at a ratio of 10 : 1. The PEG solution prepared by mixing 200 µl of RPMI1640 medium containing 10% FBS heated at 37°C and 800 µl of PEG1500 (Boehringer) was added to the cells. The solution was incubated for 5 minutes for cell fusion. Centrifugation was performed at 1700 rpm for 5 minutes to remove supernatants. Cells were suspended in 150 ml of RPMI1640 medium (HAT selective medium) containing 15% FBS, to which 2% equivalent of HAT solution (Gibco) had been added and then seeded onto fifteen 96-well plates (Nunc) at 100 µl per well. Cells were cultured for 7 days under conditions of 37°C and 5% CO₂, so that hybridomas resulting from fusion of spleen cells to myeloma cells were obtained. Hybridomas were selected using binding affinity to CAPRIN-1 protein of the antibody produced by the prepared hybridomas as an indicator. The CAPRIN-1 protein solution (1 µg/ml) was added at 100 µl per well of 96-well plates and then incubated at 4°C for 18 hours. After each well was washed 3 times with PBS-T, 0.5% Bovine Serum Albumin (BSA) solution (Sigma) was added at 400 µl per well, and then the plates were incubated at room temperature for 3 hours. The solution was removed and then each well was washed 3 times with 400 µl of PBS-T. Each culture supernatant of the hybridomas obtained above was added at 100 µl per well and then incubated at room temperature for 2 hours. After each well was washed 3 times with PBS-T, an HRP-labeled anti-mouse IgG (H+L) antibody (Invitrogen) diluted 5000-fold with PBS was added at 100 µl per well and then incubated at room temperature for 1 hour. After each well was washed 3 times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of hybridomas producing antibodies with high absorbances were selected. The selected hybridomas were added at 0.5 hybridomas per well of 96-well plates and then cultured. After 1 week, hybridomas forming a single colony in wells were observed. Cells in these wells were further cultured. Hybridomas were selected using binding affinity to CAPRIN-1 protein of the antibody produced by cloned hybridomas as an indicator. The CAPRIN-1 protein solution (1 µg/ml) was added at 100 µl per well of 96-well plates and then incubated at 4°C for 18 hours. Each well was washed 3 times with PBS-T, a 0.5% BSA solution was added at 400 µl per well, and then incubated at room temperature for 3 hours. The solution was removed and then each well was washed 3 times with 400 µl of PBS-T. Each culture supernatant of the hybridomas obtained above was added at 100 µl per well and then incubated at room temperature for 2 hours. Each well was washed 3 times with PBS-T, an HRP-labeled anti-mouse IgG (H+L) antibody (Invitrogen) diluted 5000-fold with PBS was added at 100 µl per well and then incubated at room temperature for 1 hour. Each well was washed 3 times with PBS-T, a TMB substrate solution (Thermo) was added at 100 µl per well and then incubated for 15-30 minutes, so that a color reaction was performed. After color development, 1 N sulfuric acid was added at 100 µl per well to stop the reaction. Absorbance at 450 nm and absorbance at 595 nm were measured using an absorption spectrometer. As a result, a plurality of mouse monoclonal antibodies exerting reactivity with CAPRIN-1 protein were obtained.

Reactivity of each monoclonal antibody with human cancer cells confirmed to express CAPRIN-1 protein on the cell membrane surface was further confirmed by flow cytometry. A mouse IgG control antibody exhibiting no reactivity with the cancer cells was used as a negative control. As a result of confirmation, several monoclonal antibodies were obtained which had stronger fluorescence intensity against the cancer cells than that of the mouse IgG control antibody and reacted strongly with the cell membrane surface of the cancer cells expressing CAPRIN-1 on the cell membrane surface. From among them, a monoclonal antibody against CAPRIN-1 described in WO2013/125630, which was an antibody comprising the amino acid sequence of a heavy-chain variable region shown in SEQ ID NO: 114 and the amino acid sequence of a light-chain variable region shown in SEQ ID NO: 115, was selected as a monoclonal antibody exhibiting reactivity with CAPRIN-1 protein.

CDR1 to CDR3 of the heavy-chain variable region of the antibody selected were identified. A nucleotide sequence was designed so as to be able to express a heavy-chain variable region in which framework regions comprising a human antibody sequence. This nucleotide sequence was inserted to a vector for mammalian expression having an insert of a human IgG1 heavy-chain constant region. Likewise, CDR1 to CDR3 of the light-chain variable region were identified. A nucleotide sequence was designed so as to be able to express a light-chain variable region in which framework regions comprised a human antibody sequence. This nucleotide sequence was inserted to a vector for mammalian expression having an insert of a human IgG1 light-chain constant region. These two recombinant expression vectors were introduced to mammalian cells according to a general method and then a culture supernatant containing humanized monoclonal antibody #1 (humanized antibody #1) against CAPRIN-1 was obtained.

The obtained culture supernatant containing the obtained humanized anti-CAPRIN-1 monoclonal antibody #1 was purified using Hitrap Protein A Sepharose FF (GE Healthcare Bio-Sciences) according to a general method, replaced with PBS(-), and filtered through a 0.22 µm filter (Millipore) for preparation of the filtrate.

The specific reactivity of the anti-CAPRIN-1 antibody to CAPRIN-1 protein was detected and confirmed by ELISA using CAPRIN-1 protein immobilized on a plate.

The reactivity of the anti-CAPRIN-1 antibody with cancer cells without permeation treatment of cell membranes was examined by flow cytometry to confirm that a portion of CAPRIN-1 was expressed on the cell membrane surface of cancer cells as shown in Examples given below.

It was confirmed by flow cytometry that, against all of breast cancer cells (BT-474), colon cancer cells (HT-29), lung cancer cells (QG56 and H1650), gastric cancer cells (NCI-N87), uterine cancer cells (HEC-1-A), prostate cancer cells (22Rv1), pancreatic cancer cells (Panc10.5), liver cancer cells (Hep3B), ovarian cancer cells (SKOV3), kidney cancer cells (Caki-2), brain tumor cells (U-87MG), urinary bladder cancer cells (T24), esophageal cancer cells (OE33), leukemia cells (OCI-AML5), lymphoma cells (Ramos), gallbladder cancer cells (TGBC14TKB), fibrosarcoma cells (HT-1080), and melanoma cells (G-361), which are human cancer cells confirmed to express CAPRIN-1 gene, and mouse kidney cancer cells (Renca) and mouse breast cancer cells (4T1) confirmed to express CAPRIN-1 gene, the humanized antibody #1 had stronger fluorescence intensity than that of a human IgG control antibody and rabbit IgG antibody serving as negative controls exhibiting no reactivity with the cancer cells and reacted strongly with the cell membrane surface of the cancer cells expressing CAPRIN-1.

Likewise, it was confirmed that the anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125640, WO2013/147169, WO2013/147176 and WO2015/020212 also reacted strongly with the cell membrane surface of the cancer cells.

### (Example 2) Antitumor effect of combination of anti-CAPRIN-1 antibody and immune checkpoint inhibitor

A stage 4 melanoma patient who had a previous history of treatment such as monotherapy with an immune checkpoint inhibitor anti-PD-1 antibody (nivolumab) and combination therapy with the anti-PD-1 antibody (nivolumab) and an anti-CTLA-4 antibody (ipilimumab) and had underwent continuation of tumor enlargement without receiving an effect from any of the therapies, was given a combination of the anti-PD-1 antibody (nivolumab) and "TRK-950" (anti-CAPRIN-1 antibody under clinical trial as a therapeutic agent for cancer) at a dose of 10 mg/kg. As a result of evaluation of the tumor sizes of the eye orbit, the lymph node in the armpit, the adrenal gland, and the pelvic peritoneum having metastatic foci by CT examination, the total tumor size was reduced by approximately 57% approximately 40 days after the start of administration and partial response was obtained. The tumor in the eye orbit completely disappeared in this evaluation. As a result of the same evaluation as above carried out approximately 5 months after the start of administration, the total tumor size was reduced by approximately 65%. Thus, treatment of cancer patients with a combination of an anti-CAPRIN-1 antibody and an immune checkpoint inhibitor was found to exert very strong drug efficacy which cannot be obtained in existing standard treatment.

All publications, patents and patent applications cited herein are incorporated herein by reference in their entirety.

## Claims

1. A medicament for treatment and/or prevention of cancer, comprising an antibody or a fragment thereof having an immunological reactivity with CAPRIN-1 protein, and an immune checkpoint inhibitor together or separately in combination.

2. The medicament according to claim 1, wherein the immune checkpoint inhibitor is a drug that inhibits interaction between PD-1 and PD-L1 and/or a drug that inhibits interaction between CTLA-4 and CD80 or CD86.

3. The medicament according to claim 1 or 2, wherein the immune checkpoint inhibitor is an anti-PD-1 antibody, an anti-PD-L1 antibody and/or an anti-CTLA-1 antibody.

4. The medicament according to any one of claims 1 to 3, wherein the immune checkpoint inhibitor is nivolumab.

5. The medicament according to any one of claims 1 to 4, wherein the cancer is cancer in a cancer patient who has not responded to cancer treatment with a medicament other than cancer treatment with a medicament comprising an antibody or a fragment thereof having an immunological reactivity with CAPRIN-1 protein, and an immune checkpoint inhibitor together or separately in combination.

6. The medicament according to any one of claims 1 to 5, wherein the cancer is cancer in a cancer patient who has not responded to cancer treatment with an immune checkpoint inhibitor.

7. The medicament according to any one of claims 1 to 6, wherein the antibody or the fragment thereof has an immunological reactivity with CAPRIN-1 protein having an amino acid sequence shown in any one of the even numbered SEQ ID NOs: 2 to 30, or an amino acid sequence having 80% or more sequence identity with the amino acid sequence.

8. The medicament according to any one of claims 1 to 7, wherein the antibody or the fragment thereof has an immunological reactivity with an extracellular region of a CAPRIN-1 protein present on a cancer cell surface.

9. The medicament according to any one of claims 1 to 8, wherein the antibody or the fragment thereof has an immunological reactivity with a partial polypeptide of CAPRIN-1 protein, the partial polypeptide having an amino acid sequence represented by any one of SEQ ID NOs: 31 to 35, 296 to 299, 308 and 309, or an amino acid sequence having 80% or more sequence identity with the amino acid sequence.

10. The medicament according to any one of claims 1 to 9, wherein the antibody is a monoclonal antibody or a polyclonal antibody.

11. The medicament according to any one of claims 1 to 10, wherein the antibody or a fragment thereof is any one of the following (A) to (M):
(A) an antibody or a fragment thereof comprising a heavy-chain variable region comprising complementarity determining regions of SEQ ID NOs: 36, 37 and 38 (CDR1, CDR2 and CDR3, respectively) and a light-chain variable region comprising complementarity determining regions of SEQ ID NOs: 40, 41 and 42 (CDR1, CDR2 and CDR3, respectively), and having an immunological reactivity with CAPRIN-1 protein;
(B) an antibody or a fragment thereof comprising a heavy-chain variable region comprising complementarity determining regions of SEQ ID NOs: 44, 45 and 46 (CDR1, CDR2 and CDR3, respectively) and a light-chain variable region comprising complementarity determining regions of SEQ ID NOs: 48, 49 and 50 (CDR1, CDR2 and CDR3, respectively), and having an immunological reactivity with CAPRIN-1 protein;
(C) an antibody or a fragment thereof comprising a heavy-chain variable region comprising complementarity determining regions of SEQ ID NOs: 52, 53 and 54 (CDR1, CDR2 and CDR3, respectively) and a light-chain variable region comprising complementarity determining regions of SEQ ID NOs: 56, 57 and 58 (CDR1, CDR2 and CDR3, respectively), and having an immunological reactivity with CAPRIN-1 protein;
(D) an antibody or a fragment thereof comprising a heavy-chain variable region comprising complementarity determining regions of SEQ ID NOs: 60, 61 and 62 (CDR1, CDR2 and CDR3, respectively) and a light-chain variable region comprising complementarity determining regions of SEQ ID NOs: 64, 65 and 66 (CDR1, CDR2 and CDR3, respectively), and having an immunological reactivity with CAPRIN-1 protein;
(E) an antibody or a fragment thereof comprising a heavy-chain variable region comprising complementarity determining regions of SEQ ID NOs: 170, 171 and 172 (CDR1, CDR2 and CDR3, respectively) and a light-chain variable region comprising complementarity determining regions of SEQ ID NOs: 173, 174 and 175 (CDR1, CDR2 and CDR3, respectively), and having an immunological reactivity with CAPRIN-1 protein;
(F) an antibody or a fragment thereof comprising a heavy-chain variable region comprising complementarity determining regions of SEQ ID NOs: 176, 177 and 178 (CDR1, CDR2 and CDR3, respectively) and a light-chain variable region comprising complementarity determining regions of SEQ ID NOs: 179, 180 and 181 (CDR1, CDR2 and CDR3, respectively), and having an immunological reactivity with CAPRIN-1 protein;
(G) an antibody or a fragment thereof comprising a heavy-chain variable region comprising complementarity determining regions of SEQ ID NOs: 182, 183 and 184 (CDR1, CDR2 and CDR3, respectively) and a light-chain variable region comprising complementarity determining regions of SEQ ID NOs: 185, 186 and 187 (CDR1, CDR2 and CDR3, respectively), and having an immunological reactivity with CAPRIN-1 protein;
(H) an antibody or a fragment thereof comprising a heavy-chain variable region comprising complementarity determining regions of SEQ ID NOs: 188, 189 and 190 (CDR1, CDR2 and CDR3, respectively) and a light-chain variable region comprising complementarity determining regions of SEQ ID NOs: 191, 192 and 193 (CDR1, CDR2 and CDR3, respectively), and having an immunological reactivity with CAPRIN-1 protein;
(I) an antibody or a fragment thereof comprising a heavy-chain variable region comprising complementarity determining regions of SEQ ID NOs: 146, 147 and 148 (CDR1, CDR2 and CDR3, respectively) and a light-chain variable region comprising complementarity determining regions of SEQ ID NOs: 149, 150 and 151 (CDR1, CDR2 and CDR3, respectively), and having an immunological reactivity with CAPRIN-1 protein;
(J) an antibody or a fragment thereof comprising a heavy-chain variable region comprising complementarity determining regions of SEQ ID NOs: 272, 273 and 274 (CDR1, CDR2 and CDR3, respectively) and a light-chain variable region comprising complementarity determining regions of SEQ ID NOs: 275, 276 and 277 (CDR1, CDR2 and CDR3, respectively), and having an immunological reactivity with CAPRIN-1 protein;
(K) an antibody or a fragment thereof comprising a heavy-chain variable region comprising complementarity determining regions of SEQ ID NOs: 290, 291 and 292 (CDR1, CDR2 and CDR3, respectively) and a light-chain variable region comprising complementarity determining regions of SEQ ID NOs: 293, 294 and 295 (CDR1, CDR2 and CDR3, respectively), and having an immunological reactivity with CAPRIN-1 protein;
(L) an antibody or a fragment thereof comprising a heavy-chain variable region comprising complementarity determining regions of SEQ ID NOs: 301, 302 and 303 (CDR1, CDR2 and CDR3, respectively) and a light-chain variable region comprising complementarity determining regions of SEQ ID NOs: 305, 306 and 307 (CDR1, CDR2 and CDR3, respectively), and having an immunological reactivity with CAPRIN-1 protein; and
(M) an antibody or a fragment thereof comprising a heavy-chain variable region comprising complementarity determining regions of SEQ ID NOs: 134, 135 and 136 (CDR1, CDR2 and CDR3, respectively) and a light-chain variable region comprising complementarity determining regions of SEQ ID NOs: 137, 138 and 139 (CDR1, CDR2 and CDR3, respectively), and having an immunological reactivity with CAPRIN-1 protein.

12. The medicament according to any one of claims 1 to 11, wherein the antibody or the fragment thereof is any one of the following (a) to (al):
(a) an antibody or a fragment thereof comprising a heavy-chain variable region comprising the amino acid sequence of SEQ ID NO: 39 and a light-chain variable region comprising the amino acid sequence of SEQ ID NO: 43;
(b) an antibody or a fragment thereof comprising a heavy-chain variable region comprising the amino acid sequence of SEQ ID NO: 47 and a light-chain variable region comprising the amino acid sequence of SEQ ID NO: 51;
(c) an antibody or a fragment thereof comprising a heavy-chain variable region comprising the amino acid sequence of SEQ ID NO: 55 and a light-chain variable region comprising the amino acid sequence of SEQ ID NO: 59;
(d) an antibody or a fragment thereof comprising a heavy-chain variable region comprising the amino acid sequence of SEQ ID NO: 63 and a light-chain variable region comprising the amino acid sequence of SEQ ID NO: 67;
(e) an antibody or a fragment thereof comprising a heavy-chain variable region comprising the amino acid sequence of SEQ ID NO: 68 and a light-chain variable region comprising the amino acid sequence of SEQ ID NO: 69;
(f) an antibody or a fragment thereof comprising a heavy-chain variable region comprising the amino acid sequence of SEQ ID NO: 70 and a light-chain variable region comprising the amino acid sequence of SEQ ID NO: 71;
(g) an antibody or a fragment thereof comprising a heavy-chain variable region comprising the amino acid sequence of SEQ ID NO: 72 and a light-chain variable region comprising the amino acid sequence of SEQ ID NO: 73;
(h) an antibody or a fragment thereof comprising a heavy-chain variable region comprising the amino acid sequence of SEQ ID NO: 74 and a light-chain variable region comprising the amino acid sequence of SEQ ID NO: 75;
(i) an antibody or a fragment thereof comprising a heavy-chain variable region comprising the amino acid sequence of SEQ ID NO: 76 and a light-chain variable region comprising the amino acid sequence of SEQ ID NO: 77;
(j) an antibody or a fragment thereof comprising a heavy-chain variable region comprising the amino acid sequence of SEQ ID NO: 78 and a light-chain variable region comprising the amino acid sequence of SEQ ID NO: 79;
(k) an antibody or a fragment thereof comprising a heavy-chain variable region comprising the amino acid sequence of SEQ ID NO: 80 and a light-chain variable region comprising the amino acid sequence of SEQ ID NO: 81;
(l) an antibody or a fragment thereof comprising a heavy-chain variable region comprising the amino acid sequence of SEQ ID NO: 82 and a light-chain variable region comprising the amino acid sequence of SEQ ID NO: 83;
(m) an antibody or a fragment thereof comprising a heavy-chain variable region comprising the amino acid sequence of SEQ ID NO: 84 and a light-chain variable region comprising the amino acid sequence of SEQ ID NO: 85;
(n) an antibody or a fragment thereof comprising a heavy-chain variable region comprising the amino acid sequence of SEQ ID NO: 86 and a light-chain variable region comprising the amino acid sequence of SEQ ID NO: 87;
(o) an antibody or a fragment thereof comprising a heavy-chain variable region comprising the amino acid sequence of SEQ ID NO: 88 and a light-chain variable region comprising the amino acid sequence of SEQ ID NO: 89;
(p) an antibody or a fragment thereof comprising a heavy-chain variable region comprising the amino acid sequence of SEQ ID NO: 90 and a light-chain variable region comprising the amino acid sequence of SEQ ID NO: 91;
(q) an antibody or a fragment thereof comprising a heavy-chain variable region comprising the amino acid sequence of SEQ ID NO: 92 and a light-chain variable region comprising the amino acid sequence of SEQ ID NO: 93;
(r) an antibody or a fragment thereof comprising a heavy-chain variable region comprising the amino acid sequence of SEQ ID NO: 94 and a light-chain variable region comprising the amino acid sequence of SEQ ID NO: 95;
(s) an antibody or a fragment thereof comprising a heavy-chain variable region comprising the amino acid sequence of SEQ ID NO: 96 and a light-chain variable region comprising the amino acid sequence of SEQ ID NO: 97;
(t) an antibody or a fragment thereof comprising a heavy-chain variable region comprising the amino acid sequence of SEQ ID NO: 98 and a light-chain variable region comprising the amino acid sequence of SEQ ID NO: 99;
(u) an antibody or a fragment thereof comprising a heavy-chain variable region comprising the amino acid sequence of SEQ ID NO: 100 and a light-chain variable region comprising the amino acid sequence of SEQ ID NO: 101;
(v) an antibody or a fragment thereof comprising a heavy-chain variable region comprising the amino acid sequence of SEQ ID NO: 102 and a light-chain variable region comprising the amino acid sequence of SEQ ID NO: 103;
(w) an antibody or a fragment thereof comprising a heavy-chain variable region comprising the amino acid sequence of SEQ ID NO: 104 and a light-chain variable region comprising the amino acid sequence of SEQ ID NO: 105;
(x) an antibody or a fragment thereof comprising a heavy-chain variable region comprising the amino acid sequence of SEQ ID NO: 106 and a light-chain variable region comprising the amino acid sequence of SEQ ID NO: 107;
(y) an antibody or a fragment thereof comprising a heavy-chain variable region comprising the amino acid sequence of SEQ ID NO: 108 and a light-chain variable region comprising the amino acid sequence of SEQ ID NO: 109;
(z) an antibody or a fragment thereof comprising a heavy-chain variable region comprising the amino acid sequence of SEQ ID NO: 110 and a light-chain variable region comprising the amino acid sequence of SEQ ID NO: 111;
(aa) an antibody or a fragment thereof comprising a heavy-chain variable region comprising the amino acid sequence of SEQ ID NO: 112 and a light-chain variable region comprising the amino acid sequence of SEQ ID NO: 113;
(ab) an antibody or a fragment thereof comprising a heavy-chain variable region comprising the amino acid sequence of SEQ ID NO: 114 and a light-chain variable region comprising the amino acid sequence of SEQ ID NO: 115;
(ac) an antibody or a fragment thereof comprising a heavy-chain variable region comprising the amino acid sequence of SEQ ID NO: 116 and a light-chain variable region comprising the amino acid sequence of SEQ ID NO: 117;
(ad) an antibody or a fragment thereof comprising a heavy-chain variable region comprising the amino acid sequence of SEQ ID NO: 118 and a light-chain variable region comprising the amino acid sequence of SEQ ID NO: 119;
(ae) an antibody or a fragment thereof comprising a heavy-chain variable region comprising the amino acid sequence of SEQ ID NO: 120 and a light-chain variable region comprising the amino acid sequence of SEQ ID NO: 121;
(af) an antibody or a fragment thereof comprising a heavy-chain variable region comprising the amino acid sequence of SEQ ID NO: 122 and a light-chain variable region comprising the amino acid sequence of SEQ ID NO: 123;
(ag) an antibody or a fragment thereof comprising a heavy-chain variable region comprising the amino acid sequence of SEQ ID NO: 124 and a light-chain variable region comprising the amino acid sequence of SEQ ID NO: 125;
(ah) an antibody or a fragment thereof comprising a heavy-chain variable region comprising the amino acid sequence of SEQ ID NO: 126 and a light-chain variable region comprising the amino acid sequence of SEQ ID NO: 127;
(ai) an antibody or a fragment thereof comprising a heavy-chain variable region comprising the amino acid sequence of SEQ ID NO: 128 and a light-chain variable region comprising the amino acid sequence of SEQ ID NO: 129;
(aj) an antibody or a fragment thereof comprising a heavy-chain variable region comprising the amino acid sequence of SEQ ID NO: 130 and a light-chain variable region comprising the amino acid sequence of SEQ ID NO: 131;
(ak) an antibody or a fragment thereof comprising a heavy-chain variable region comprising the amino acid sequence of SEQ ID NO: 132 and a light-chain variable region comprising the amino acid sequence of SEQ ID NO: 133; and
(al) an antibody or a fragment thereof comprising a heavy-chain variable region comprising the amino acid sequence of SEQ ID NO: 300 and a light-chain variable region comprising the amino acid sequence of SEQ ID NO: 304.

13. The medicament according to any one of claims 1 to 12, wherein the antibody is a human antibody, a humanized antibody, a chimeric antibody or a single chain antibody.

14. The medicament according to any one of claims 1 to 13, wherein the cancer is cancer expressing CAPRIN-1 protein on a cell membrane surface.

15. The medicament according to any one of claims 1 to 14, wherein the cancer is melanoma, breast cancer, kidney cancer, colon cancer, pancreatic cancer, lung cancer, renal cell carcinoma, Hodgkin's lymphoma, head and neck cancer, gastric cancer, mesothelioma, colorectal cancer, esophageal cancer, gastroesophageal junction cancer, hepatocellular carcinoma, glioblastoma, urothelial carcinoma, ovarian cancer, urinary bladder cancer, uterine cancer, primary central nervous system lymphoma, primary testicular lymphoma, biliary tract cancer, bile duct cancer, brain tumor, prostate cancer, leukemia, lymphoma, liver cancer, sarcoma, fibrosarcoma, mastocytoma, adrenocortical carcinoma, Ewing's tumor, multiple myeloma, testicular cancer, thyroid cancer, basal cell carcinoma, Paget's disease or skin cancer.

16. An agent increasing drug efficacy of a pharmaceutical composition for treatment and/or prevention of cancer comprising an antibody or a fragment thereof having an immunological reactivity with CAPRIN-1 protein as an active ingredient, wherein the agent comprises an immune checkpoint inhibitor as an active ingredient.

17. An agent increasing drug efficacy of a pharmaceutical composition for treatment and/or prevention of cancer comprising an immune checkpoint inhibitor as an active ingredient, wherein the agent comprises an antibody or a fragment thereof having an immunological reactivity with CAPRIN-1 protein as an active ingredient.

18. A method for treating and/or preventing cancer, comprising administering an antibody or a fragment thereof having an immunological reactivity with CAPRIN-1 protein, and an immune checkpoint inhibitor together or separately to a subject.
